# EUROPÄISCHE PATENTSCHRIFT

(11) **EP 0 329 008 B1**
(45) Veröffentlichungstag und Bekanntmachung des Hinweises auf die Patenterteilung: **11.08.1999**
(21) Anmeldenummer: 89102198.2
(22) Anmeldetag: 09.02.1989
(51) Int. Cl.: C07D 501/34, A61K 31/545

(54) **Cephalosporinderivative und Verfahren zu ihrer Herstellung**
Cephalosporinderivatives and process for its preparation
Dérivés de céphalosporine et procédé pour leur préparation

(30) Priorität: 17.02.1988 DE 3804841
(43) Veröffentlichungstag der Anmeldung: 23.08.1989
(73) Patentinhaber: HOECHST AKTIENGESELLSCHAFT, 65926 Frankfurt am Main (DE)
(72) Erfinder: Adam, Friedhelm Dr., D-6238 Hofheim am Taunus (DE); Blumbach, Jürgen, Dr., Bombay 400 006 (IN); Dürckheimer, Walter, Dr., D-6234 Hattersheim am Main (DE); Fischer, Gerd, Dr., D-6230 Frankfurt am Main 80 (DE); Mencke, Burghard, Dr., D-5409 Holzappel (DE); Isert, Dieter, Dr., D-6236 Eschborn (DE); Seibert, Gerhard, Prof. Dr., D-6100 Darmstadt (DE)

(56) Entgegenhaltungen:
- EP-A- 0 034 536
- EP-A- 0 049 119

## Beschreibung

Die Erfindung betrifft neue Cephalosporinderivate, die besonders für die orale Applikation geeignet sind, ein Verfahren zu ihrer Herstellung und pharmazeutische Zubereitungen, die solche Verbindungen enthalten.

Obwohl viele klinische relevante Cephalosporine mit breitem antibakteriellem Spektrum entwickelt worden sind, eignen sich die meisten von ihnen nur für eine parenterale Verabreichung, da sie nach oraler Gabe, wenn überhaupt, nur sehr unzureichend resorbiert werden. In vielen Fällen ist es jedoch wünschenswert, dem Patienten hochwirksame Antibiotika in oraler Form zu geben.

Die bislang bekannten Cephalosporin-Antibiotika erfüllen nicht alle Anforderungen, die an ein solches Medikament gestellt werden müssen, nämlich eine hohe antibakterielle Aktivität gegen grampositive (speziell Staphylokokken) und gramnegative Erreger und gleichzeitig eine gute Resorption im Magen-Darm-Trakt.

In einigen Fällen ist es gelungen, die Resorption eines Cephalosporins im Gastrointestinaltrakt durch Veresterung der 4-Carboxylgruppe zu erhöhen. Da die Cephalosporinester, in der Regel per se keine antibiotische Wirksamkeit aufweisen, muß die Esterkomponente so gewählt werden, daß der Ester nach der Resorption durch körpereigene Enzyme, wie Esterasen, wieder rasch und vollständig zum Cephalosporin mit freier Carboxygruppe zurückgespalten wird.

Das Ausmaß der enteralen Resorption von Cephalosporin ist maßgeblich abhängig von der chemischen Struktur des Cephalosporins und der jeweiligen Esterkomponente. Schon kleine strukturelle Variationen am Cephalosporin-Grundgerüst oder in der Esterkomponente können die Resorption beeinflussen. Das Auffinden geeigneter Komponenten ist rein empirisch.

So führt beispielsweise die Einführung eines sauren Substituenten in der 7β-Seitenkette von Aminothiazolyl-Cephalosporinen, wie z.B. im Cefixime, zu einer enteral resorbierenden Verbindungen, während Verbindungen mit neutralen Seitenktten, wie z.B. im Cefuroxim, nur in Form von Prodrug-Estern enteral resorbiert werden. Die Dosis-Wirkungs-Proportionalität ist dabei oft nicht linear und die erzielten therapeutischen Serumspiegel sind nicht befriedigent. Weitere Ester aus der Reihe der Aminothiazolyl-Cephalosporine werden beispielsweise in den EP-A 29 557, 34 536, 49 119 und 134 420 erwähnt.

Wir fahnden nun durch systematisch durchgeführte in vivo-Untersuchungen in verschiedenen Tierspecies eine enge Gruppe von oral applizierbaren Ceph-3-em-4-carbonsäureestern, die eine ausreichende chemische Stabilität besitzen und durch ausgewogene Lipid- und Wasserlöslichkeit rasch und in therapeutisch beachtlichem Maße im Magen-Darm-Trakt resorbiert werden.

Gegenstand der Erfindung sind demnach Diastereomeres I der 7-[2-(2-Aminothiazol-4-yl)-2-(z)-hydroxyiminoacetamido]-3-(methoxymethyl)-3-cephem-4-carbonsäure-[a-(2,2-dimethylpropionyloxy)-ethyl]-esters der Formel I 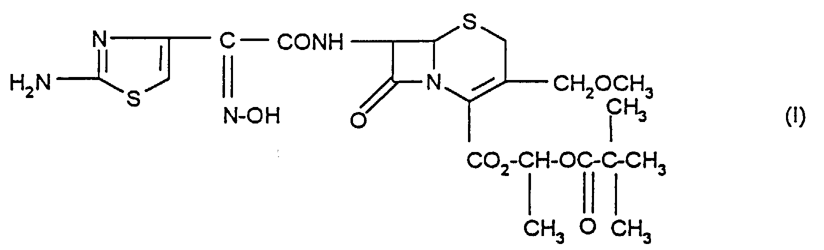 worin die HO-Gruppe in Syn-Position steht und deren physiologisch verträgliche Säureadditionssalze.

Als physiologisch verträgliche Säureadditionssalze kommen die für Cephalosporin-Antibiotika bekannten Salze in Betracht, wie z.B. das Hydrochlorid, Sulfat, Maleinat, Citrat, Acetat oder Formiat. Ihre Herstellung erfolgt in an sich bekannter Weise durch Zusammengeben der Komponenten in einem wäßrigen oder organischen Lösungsmittel oder in einer geeigneten Lösungsmittelmischung.

Die Verbindungen der Formel I besitzen im Esterteil ein chirales Zentrum. Bei Verwendung racemischer Verbindungen der allgemeinen Formel III liegen die Cephemcarbonsäureester der allgemeinen Formel I als ein Gemisch von zwei Diastereomeren vor, die nach bekannten Methoden in die beiden Einzelkomponenten trennbar sind.

Gegenstand der Erfindung ist weiterhin ein Verfahren zur Herstellung von Diastereomers I des 7-[2-(2-Aminothiazol-4-yl)-2-(Z)-hydroxyimino-acetamido]-3-(methoxymethyl)-3-cephem-4-carbonsäure-[α-(2,2-dimethylpropionyloxy)-ethyl]-esters der Formel I 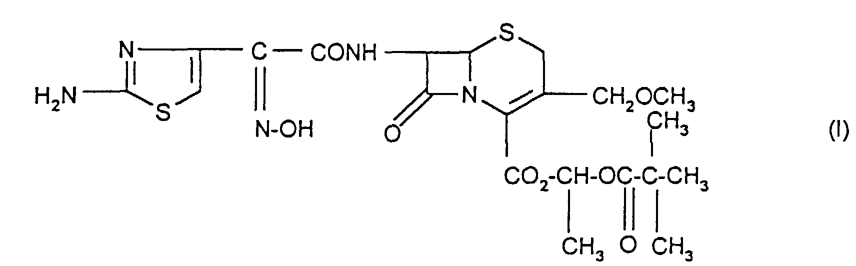 worin die HO-Gruppe in Syn-Position steht und von deren physiologisch verträglichen Säureadditionssalzen, das dadurch gekennzeichnet ist, daß man
(a) eine Verbindung der Formel II 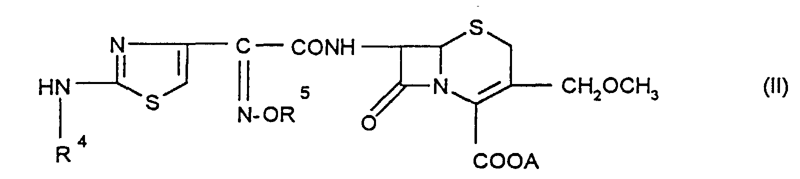 in der R⁴ für eine Aminoschutzgruppe, R⁵ eine leicht abspaltbare Gruppe und A ein Kation steht, mit einer Verbindung der allgemeinen Formel III 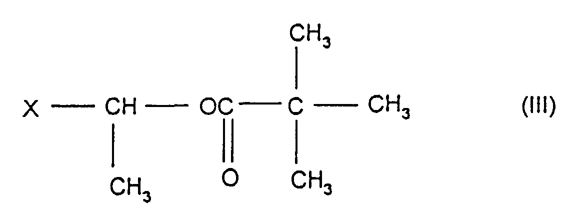 worin X für eine Abgangsgruppe steht, umsetzt zu dem Diastereomerengemisch der Verbindung der Formel IV 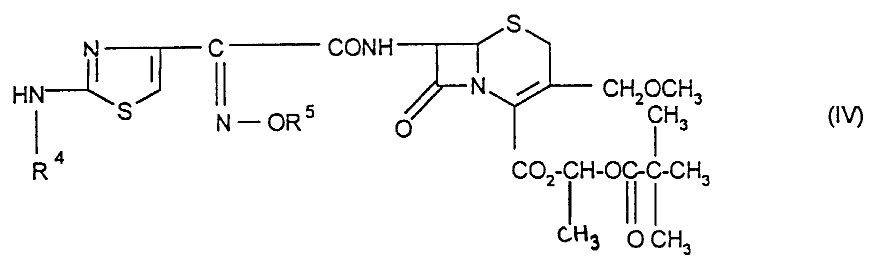 worin R⁴ und R⁵ die oben genannte Bedeutung haben und anschließend auf chromatographischem Wege die Diastereomeren voneinander trennt, oder
(b) eine Verbindung der allgemeinen Formel V 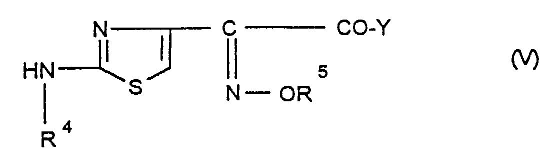 in der R⁴ und R⁵ die vorstehende Bedeutung besitzen und Y für eine aktivierende Gruppe steht, mit einer Verbindung der Formel VI 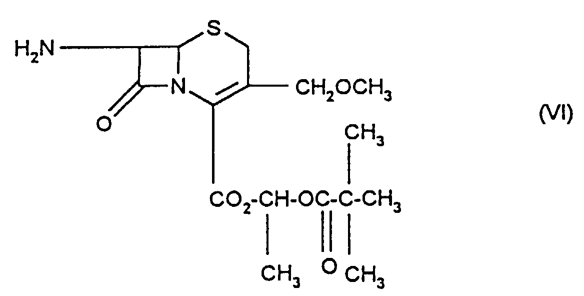 oder mit einem Salz dieser Verbindung, zu dem Diastereomerengemisch der Verbindung der Formel IV umsetzt, und anschließend auf chromatographischem Weg die Diastereomeren voneinander trennt, und
(c) die Gruppen R⁴ und R⁵ vom weniger polaren Diastereomeren der Formel IV durch Behandlung mit Trifluoressigsäure, Ameisensäure oder verdünnter Salzsäure abspaltet.

In den allgemeinen Formeln II, IV und V steht R⁴ für eine aus der Peptid- und Cephalosporinchemie bekannte Aminoschutzgruppe, vorzugsweise für Formyl, Chloracetyl, Bromacetyl, Trichloracetyl, Benzyloxycarbonyl, tert.-Butoxycarbonyl oder Trityl und R⁵ für eine ebenfalls aus der Peptid- und Cephalosporinchemie bekannte leicht abspaltbare Gruppe, vorzugsweise for Benzhydryl, Trityl, Tetrahydropyranyl oder 1-Methoxy-1-methyl-ethyl. Besonders bevorzugt für R⁴ ist Trityl, für R⁵ Trityl und 1-Methoxy-1-methyl-ethyl.

In Formel III hat X die Bedeutung einer für Veresterungen allgemein bekannten Abgangsgruppe, wie beispielsweise Chlor, Brom, Jod, Phenylsulfonyloxy, p-Toluolsulfonyloxy oder Methylsulfonyloxy, vorzugsweise Chlor, Brom oder Jod, insbesondere Jod.

Als Base, die dem Kation A in der allgemeinen Formel II zugrundeliegen, seien z.B. genannt Natriumhydrogencarbonat, Kaliumhydrogencarbonat, Natriumcarbonat, Kaliumcarbonat, gegebenenfalls substituierte, alkylierte Aminbasen, wie z.B. Trimethylamin, Triethylamin, Diisopropylamin, Ethyldiisopropylamin, N,N-Dimethylanilin, N,N-Dimethylbenzylamin, 1,5-Diazabicyclo[4,3,0]non-5-en (DBN), 1,8-Diazabicyclo[5,4,9]undec-7-en (DBU), Pyridin, Picolin oder 2,6-Dimethylpyridin. Bevorzugte Basen sind Natrium- oder Kaliumhydrogencarbonat, Natrium- oder Kaliumcarbonat, Triethylamin, N,N-Dimethylanilin, DBN oder DBU.

Durch Umsetzung der freien Carbonsäuren mit diesen Basen erhält man die Salze der allgemeinen Formel II, in der A für ein Kation, wie beispielsweise Natrium oder Kalium, aber auch für Magnesium oder Calcium oder für ein gegebenenfalls substituiertes alkyliertes Ammoniumion, wie beispielsweise Ammonium, Trimethylammonium, Triethylammonium, Tetrabutylammonium, Diisopropylammonium, Ethyldiisopropylammonium, Diazabicyclo[0,3,4]nonenium oder Diazabicyclo[0,4,5]undecium steht. Bevorzugte Bedeutungen von A sind Natrium, Kalium, Triethylammonium, N,N-Dimethylanilinium, sowie das DBN- und DBU-ion.

Die Umsetzung der Verbindungen der Formel II mit den Verbindungen der Formel III kann einem organischen Lösungsmittel bei etwa -20 bis etwa +50°C, bevorzugt bei etwa 0°C bis Raumtemperatur durchgeführt werden. Als Lösungsmittel können beispielsweise dienen Ketone, wie beispielsweise Aceton oder Methylethylketon, N,N-Dimethylformamid (DMF), N,N-dimethylacetamid (DMA), N-Methylpyrrolidon oder Dimethylsulfoxid (DMSO). Bevorzugt sind DMF, DMA, N-Methylpyrrolidon und DMSO. Besonders bevorzugt ist DMF.

Die Abspaltung der Gruppen R⁴ und R⁵ aus den erhaltenen Verbindungen der Formel IV erfolgt in aus der Peptid- und Cephalosporinchemie an sich bekannter Weise, z.B. mit Trifluoressigsäure, verdünnter Salzsäure, vorzugsweise mit Ameisensäure unter Zugabe von etwas Wasser.

Setzt man eine Verbindung der Formel V mit einer Verbindung der Formel VI um, so stellt Y eine die Carboxylgruppe aktivierende Gruppe dar, wie sie aus der Peptid- und Cephalosporinchemie für entsprechende Reaktionen bekannt ist, beispielsweise ein Halogenid, vorzugsweise Chlorid, eine aktivierende Estergruppe, beispielsweise mit 1-Hydroxybenzotriazol oder ein gemischtes Anhydrid, beispielsweise mit Benzolsulfonsäure oder Toluolsulfonsäure. Die Aktivierung der Carboxylgruppe ist auch in literaturbekannter Weise über die Zugabe eines Kondensationsmittels, wie z.B. eines Carbodiimids, möglich.

Die Verbindung der allgemeinen Formel VI läßt sich als solche oder in Form eines Salzes, beispielsweise des Tosylats, Hydrochlorids oder Hydrojodids einsetzen, wobei die Verwendung kristalliner Salze im Hinblick auf die Reinheit der Produkte vorteilhaft sein kann.

Die Umsetzung von Verbindungen der Formel V mit solchen der Formel VI kann in einem organischen Lösungsmittel, wie beispielsweise Methylenchlorid, Chloroform, Aceton, Methylethylketon, Dimethylformamid, Dimethylacetamid oder Wasser, oder auch in Mischungen dieser Lösungsmittel erfolgen.

Die Acylierungsreaktion kann zweckmäßigerweise bei Temperaturen von etwa -50°C bis etwa +50°C, vorzugsweise -40°C bis +30°C, gewünschtenfalls in Anwesenheit einer Base, wie beispielsweise Triethylamin oder Pyridin, durchgeführt werden. Der Basenzusatz dient dazu, die bei der Kondensation freiwerdende acide Komponente zu binden.

Die Ausgangsverbindungen der Formel III können in an sich bekannter Weise hergestellt werden, indem man Verbindungen der Formel 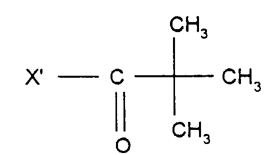 in der X' für eine Abgangsgruppe steht, mit einem Aldehyd der Formel

CH₃ - CHO

umsetzt. Die bevorzugte Bedeutung von X' ist Brom oder Chlor. Die Reaktion wird zeckmäßigerweise in einem organischen Lösungsmittel, wie einem halogenierten Kohlenwasserstoff, beispielsweise Methylenchlorid oder Chloroform, in Anwesenheit eines Katalysators, wie beispielsweise Zinkchlorid oder Aluminiumchlorid bei einer Temperatur von zweckmäßigerweise -10°C bis +10°C durchgeführt.

Die Ausgangsverbindungen der Formel III, in der X für Chlor steht, können alternativ hergestellt werden, indem man eine Carbonsäure der Formel 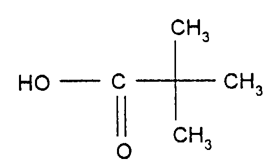 deren Herstellung in J. Amer. Chem. Soc. 70, S. 1153 beschrieben wird, mit einer Verbindung der Formel 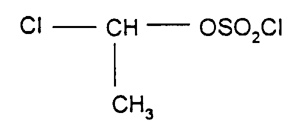 deren Herstellung in Synthetic Communications 14, S. 857 beschrieben wird, in Gegenwart einer Base wie beispielsweise Natriumcarbonat, Kaliumcarbonat, Natriumhydrocarbonat oder Kaliumhydrogencarbonat, bevorzugt Natriumhydrogencarbonat, umsetzt. Die Reaktion wird bevorzugt bei 0°C bis Raumtemperatur in einem Zweiphasengemisch, vorzugsweise bei aus Wasser und einem chlorierten Kohlenwasserstoff, wie z.B. Methylchlorid oder Chloroform in Anwesenheit eines Phasentransfer-Katalysators, wie beispielsweise Tetrabutylammoniumhydrogensulfat, durchgeführt.

Ausgangsverbindungen der Formel III können auch durch Halogenaustausch hergestellt werden. So erhält man beispielsweise eine Verbindung der Formel III, in der X für Jod steht, durch Umsetzen der entsprechenden Verbindungen III, in der X für Chlor oder Brom steht, mit einem Jodidsalz, wie beispielsweise Natriumjodid.

Die Herstellung der Ausgangsverbindungen der allgemeinen Formel II ist in der EP-PS 34 356 beschrieben.

Die Herstellung von Ausgangsverbindungen der allgemeinen Formel V mit der aktivierten Carboxylgruppe erfolgt in literaturbekannter Weise, die zu den Verbindungen der Formel VI führende Veresterung auf diesselbe Weise, wie sie für die Herstellung der Ester der allgemeinen Formel VI beschrieben wurde.

Die Ceph-3-em-4-carbonsäureester der allgemeinen Formel I besitzen eine Reihe von physikochemischer und biologischer Eigenschaften, die sie zu wertvollen Cephalosporin-Antibiotika zur oralen Verabreichungen machen. Sie sind stabile, farblose und in gängigen organischen Lösungsmitteln gut lösliche Verbindungen, die im Darm resorbiert, im Serum rasch zum antibiotisch aktiven Cephalosporin-Derivat der Formel 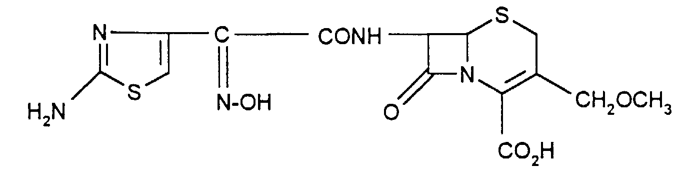 gespalten werden und sich daher hervorragend zur Behandlung von bakteriellen Infektionskrankheiten, wie z.B. der Infektion der Atemwege oder des Urogenitaltraktes eignen.

Die erfindungsgemäßen Verbindungen werden oral verabreicht in Form von üblichen pharmazeutischen Zubereitungen, wie z.B. Kapseln, Tabletten, Pulvern, Sirupen oder Suspensionen. Die Dosis hängt ab vom Alter, den Symptomen und dem Körpergewicht des Patienten sowie von der Dauer der Behandlung. Sie liegt jedoch in der Regel zwischen etwa 0,2 g und etwa 5 g täglich, vorzugsweise zwischen etwa 0,5 g und etwa 3 g täglich. Die Verbindungen werden vorzugsweise in aufgeteilten Dosen verabreicht, beispielsweise 2 bis 4-mal täglich, wobei die Einzeldosis beispielsweise zwischen 50 und 500 mg Wirkstoff enthalten kann.

Die oralen Zubereitungen können die üblichen Trägerstoffe und/oder Verdünnungsmittel enthalten. So kommen beispielsweise für Kapseln oder Tabletten in Betracht Bindemittel, wie z.B. Gelatine, Sorbitol, Polyvinylpyrrolidon oder Carboxymethylcellulose, Verdünnungsmittel, wie z.B. Lactose, Zucker, Stärke, Calciumphosphate oder Polyethylenglykol, Gleitstoffe, wie z.B. Talkum oder Magnesiumstearat, für flüssige Zubereitungen, z.B. wäßrige oder ölige Suspensionen, Sirupe oder ähnliche bekannte Zubereitungsformen.

Die folgenden Beispiele dienen zur weiteren Erläuterung der Erfindung, schränken sie jedoch nicht darauf ein.

### A. Herstellung von Ausgangssubstanzen

### Herstellungsbeispiel 1

### 1 -Chlorethyl-chlorschwefelsäureester

18 ml Chlorschwefelsäure werden bei 0°C innerhalb von 15 Minunten zu 36 g Chlormeisensäure-1-chlorethylester zugetropft. Das Gemisch wird solange heftig gerührt, bis die Gasentwicklung aufgehört hat (etwa 4 Stunden). Es wird auf 500 ml Eiswasser/Methylenchlorid (1:1) geschüttet, die organische Phase abgetrennt und die wäßrige Phase noch zweimal mit je 30 ml Methylenchlorid extrahiert. Die vereinigten organischen Phasen werden zweimal mit je 300 ml gesättigter Natriumhydrogencarbonat-Lösung und einmal mit 300 ml gesättigter Natriumchlorid-Lösung gewaschen, über Magnesiumsulfat getrocknet, eingeengt und der Rückstand im Vakuum destilliert.
Kp (10) : 49 - 50°C; Ausbeute 20 g.

### Herstellungsbeispiel 2

### α-(2,2-Dimethyl-propionsäure-jodethylester)

Zu einer Lösung von 7,5 g (50 mmol) Natriumjodid in 50 ml wasserfreiem Acetonitril wurden bei 0°C, 2,8 ml (50 mmol) Acetaldehyd in 10 ml wasserfreiem Acetonitril zugetropft. Anschließend gab man 6, 2 ml (50 mmol) 2,2-Dimethylpropionsäurechlorid in 10 ml Acetonitril zu und rührte 2 Stunden bei 0°C nach. Die gelbliche Lösung wurde danach auf 200 ml Eiswasser gegeben und mit 250 ml Pentan extrahiert. Die organische Phase wurde je einmal mit Wasser, 10 %iger Natriumbisulfitlösung und gesättigter Kochsalzlösung gewaschen und über Magnesiumsulfat getrocknet. Nach Abzug des Lösungsmittels im Vakuum (20°C Badtemperatur) erhielt man 7,9 g eines gelblichen Öls.

¹H-NMR (60 MHz, CDCl₃): δ (ppm) : 1.25 (9H, s. tert.-Butyl), 2,15 (3H, q, CH-CH₃), 6.8 (1H, q, CH-CH₃).

### Herstellungsbeispiel 3

### 7-[2-(2-Tritylaminothiazol-4-yl)-2-(Z)-trityl-oximinoacetamido]-3-(methoxymethyl)-3-cephem-4-carbonsäure

### Stufe 1

### 2-(2-Tritylaminthioazol-4-yl)-2-(Z)-trityloximino-essigsäurechlorid

46,5 g (60 mmol) 2-(2-Tritylaminothiazol-4-yl)-2-(Z)-trityloximino-essigsäure-triethylammoniumsalz wurden in 400 ml wasserfreiem Methylenchlorid gelöst und auf -70°C abgekühlt. Dazu tropft man eine Lösung von 12,3 g (60 mmol) Phosphorpentafluorid in 200 ml wasserfreim Methylenchlorid so zu, daß die Temperatur nicht über -60°C steigt. Danach rührte man noch 1 Stunde bei dieser Temperatur nach und engte danach die Lösung umgehend im Vakuum ein. Es wurde noch zweimal mit je 100 ml Methylenchlorid versetzt und einrotiert.

### Stufe 2

### 7-[2-(2-Tritylaminothiazol-4-yl)-2-(Z)-trityl-oximinoacetamido]-3-(methoxymethyl)-3-cephem-4-carbonsäure

Eine Lösung des Produktes aus Stufe 1 in 50 ml Methylenchlorid und 400 ml Aceton wurde zu einer auf 0°C gekühlten Lösung von 18 g 7-Amino-3-methoxymethyl-ceph-3-em-4-carbonsäure in 400 ml Aceton/Wasser (1:1), der zuvor 5,6 g (66 mol) Natriumhydrogencarbonat und 18 ml (132 mmol) Triethylamin zugesetzt wurden, getropft. Nach 2 Stunden Rühren bei 0°C wurde mit 1N Salzsäure auf pH 4 gestellt und mit der doppelten Menge Essigester extrahiert. Anschließend wurden die Phasen getrennt und die organische Lösung mit 5 %iger Kaliumhydrogensulfat-Lösung extrahiert. Nach Trocknen über Magnesiumsulfat wurde eingeengt, der Rückstand in 150 ml Methylenchlorid gelöst und unter kräftigem Rühren in 1,8 I Diisopropylether eingetropft. Nach Absaugen erhielt man 45,7 g (85 %) der Titelverbindung.

### B. Ausführungsbeispiele

### Beispiel 1

### 7-[2-(2-Aminothiazol-4-yl)-2-(Z)-hydroxyimino-acetamide]-3-(methoxymethyl)-3-cephem-4-carbonsäure-α-carbonsäure-α-(2,2-dimethylpropionyloxy)-ethylester

### Verfahrensvariante a)

### Stufe 1

### 7-[2-(2-Tritylaminothiazol-4-yl)-2-(Z)-trityl-oximinoacetamido]-3-(methoxymethyl)-3-cephem-4-carbonsäure-α-carbonsäure-α-(2,2-dimethyl-pronionyloxy)-ethylester

Eine Lösung von 14 g (15,6 mmol) 7-[2-(2-Tritylaminothiazol-4-yl)-2-(Z)-trityloximinoacetamido]-3-(methoxymethyl)-3-cephem-4-carbonsäure in 300 ml wasserfreiem Dimethylformamid wurden mit 1,07 g (7,7 mmol) Kaliumcarbonat versetzt und bis zur Auflösung des Salzes bei Raumtemperatur gerührt. Danach kühlte man in Eisbad und gab 5,1 g α-2,2-Dimethyl-propionsäure-jodethylester zu. Man rührte noch 2 Stunden bei 0°C nach, zog das Lösungsmittel im Vakuum ab und verteilte zwischen Essigester und Wasser. Die organische Phase wurde über Magnesiumsulfat getrocknet, die Lösung eingeengt und der feste Rückstand chromatographiert (SiO₂; Toluol/EE = 10 + 1). Man erhielt 4,0 g reine Titelverbindung als Gemisch der beiden Diastereomeren.
¹H-NMR (270 MHz, CDCl₃) : δ (ppm) = 1.22 (9H, s, C(CH₃)₃, 1.55 (3H, d, CH₃), 3.3 (3H, s, OCH₃), 3.4 (2H, AB, J = 16 Hz, SCH₂), 4.30 (2H, s, CH₂OCH₃), 5.05 (1H, d-6H), 6.08 (1H, m, 7-H), 6.4 (1H, breites s, Thiazol-H), 6.9 (1H, q, OCH-CH₃), 7.1-74 (32H, aromat. H, 2 x NH).

### Stufe 2

### 7-[2-(2-Aminothiazol-4-yl)-2-(Z)-hydroimino-acetamido]-3-methoxymethyl)-3-cephem-4-carbonsäure-α-(2,2-dimethyl-propionyloxy)-ethylester

4,0 g der in Stufe 1 erhaltenen Verbindung wurden bei Raumtemperatur in 60 ml Ameisensäure gelöst, mit 15 ml Wasser versetzt und 30 Minuten bei Raumtemperatur gerührt. Nach beendeter Reaktion saugte man ab, engte die Lösung ein, versetzte mit Toluol und destillierte das Lösungsmittel ab. Den harzartigen Rückstand rührte man in einem Gemisch aus Diisopropylether/Diethylether aus. Man erhielt 2,2 g der gewünschten Titelverbindung als Gemisch der beiden Diastereomeren.

¹H-NMR (270 MHz, DMSO-d₆): δ (ppm) = 1.5 (9H, 2 x s, tert.-Butyl), 1.5 (3H, dd, CH-CH₃), 3.2 (3H, 2 x s, OCH₃), 3.55 (2H, m, S-CH₂-), 4.15 (2H, d, CH₂-OCH₃), 5.2 (1H, dd, J = 6 Hz, H-6), 5.85 (1H, m, H-7), 6.65 (1H, s, Thiazol-H), 6.9 (1H, m, -O-CH-O), 7.1 (2H, breites s, NH₂), 9.45 (1H, d, J = 8 Hz, -NH), 11.3 (1H, s, Oxim-H).

### Verfahrensvariante b)

### Vorstufe

### 2-(2-Tritylaminothiazol-4-yl)-2-(Z)-(1-methyl-1-methoxy)-ethoxy-imino-essigsäure-p-toluolsulfonsäureanhydrid

Zu einer Suspension von 6 g (10 mmol) 2-(2-Tritylaminothiazol-4-yl)-2-(Z)-(1-methyl-1-methoxy)ethoxyiminoessigäsure-triethylammoniumsalz in 30 ml Aceton wurden 2,1 g (11 mmol) p-Toluolsulfonsäurechlorid gegeben und 1,5 Stunden bei Raumtemperatur gerührt. Anschließend versetzte man mit 40 ml Diethylether, kühlte auf -10°C und saugte danach ab. Das Produkt wurde noch dreimal mit je 20 ml Ether nachgewaschen und getrocknet. Man erhielt 10 g Produkt, das aus einem Gemisch der Titelverbindung und Triethylamin-hydrochlorid bestand und ohne weitere Reinigung weiterverarbeitet wurde.

### Stufe 1

7-[2-(2-Tritylaminothiazol-4-yl)-2-(Z)-(1-methyl-1-methoxy)-ethoxyiminoacetamido]-3-(methoxymethyl)-3-cephem-4-carbonsäure-α-(2,2-dimethylpropionlyoxy)-ethylester.

0,73 g (3 mmol) 7-Amino-3-methoxymethyl-ceph-3-em-4-carbonsäure wurden in 30 ml Methylenchlorid suspendiert und mit 0,37 ml (2,4 mmol) 1,8-Diazabicyclo-(5,4,0)-undec-7-en (DBU) bei 0°C versetzt. Die entstandene Lösung wurde noch 30 Minuten nachgerührt, anschließend 0,79 g (3,3 mmol) α-2,2-Dimethylpropionsäure-jodethylester (Herstellungsbeispiel 4) zugetropft und 2 Stunden bei dieser Temperature nachgerührt. Danach gab man 1,9 g (2,4 mmol) des in der Vorstufe erhaltenen gemischten Anhydride zu und hielt die Lösung 1,5 Stunden bei 0°C.

Nach beendeter Reaktion wurde das Lösungsmittel im Vakuum abgezogen und der Rückstand chromatographiert (SiO₂; Toluol/Essigester = 1:1). Man erhielt 1,1 g (62 %) der gewünschten Verbindung als Gemisch der beiden Diastereomeren.

¹H-NMR (270 MHz, DMSO-d₆): δ (ppm) = 1.15 (9H, 2 x s, tert.-Butyl), 1.36 (6H, s, O-(CH₃)₂, 1,51 (3H, dd, CH-CH₃), 3.1 (3H, s, C(CH₃)₂-OCH₃), 3.20 (3H, s, OCH₃), 3.58 (2H, AB, J = 16 Hz, SCH₂-), 4.12 (2H, d, CH₂OCH₃), 5.18 (1H, AB, J = 8 Hz, 6-H), 5.85 (1H, m 7-H), 6.70 (1H, s, Thiazol-H), 6.9 (1H, m, -OCO-O), 7.15 - 7.4 (16H, m, aromat. H und NH), 9.5 (1H, d, -NHCO).

### Stufe 2

7-[2-(2-Aminothiazol-4-yl)-2-[Z)-hydroxyimino-acetamido]-3-(methoxymethyl)-3-cephem-4-carbonsäure-α-carbonsäure-α-(2,2-dimethylpropionyloxy)-ethylester 1 g (1.2 mmol) der in Stufe 1 erhaltenen Verbindung wurden in 15 ml Ameisensäure gelöst und anschließend mit 3 ml Wasser versetzt. Nach 1 Stunde bei 0°C wurde vom entstandenen Triphenylcarbinol abgesaugt und das Filtrat im Vakuum eingeengt. Der Rückstand wurde mehrmals mit Toluol versetzt und erneut einrotiert. Anschließend nahm das Rohprodukt in Essigester auf, extrahiert mit gesättigter Natriumhydroncarbonatlösung, trocknete die organische Phase über Magnesiumsulfat und rotierte ein. Den Rückstand verrieb man mit Diethylether. Man erhielt 340 g (57 %) der Titelverbindung, die in allen Eigenschaften mit dem nach Verfahrensvariante a) erhaltenen Produkt identisch ist.

In Stufe 1 kann anstelle des Cephemcarbonsäureesters auch dessen Tosylat eingesetzt werden, das sich folgendermaßen herstellen läßt.

Zu einer Suspension von 2,44 g (10 mmol) 7-Amino-3-methoxymethyl-3-cephem-4-carbonsäure in 100 ml wasserfreiem Methylenchlorid wurden 1,5 ml (10 mmol) 1,8-Diazabicyclo(5,4,0)-undec-7-en (DBU) gegeben und die entstehende Lösung 30 Minuten bei Raumtemperatur nachgerührt. Anschließend wurden bei 0°C, 2,6 g (10 mmol) a-2,2-Dimethyl-propionäsure-jodethylester zugetropft und bei Raumtemperatur 1 Stunde nachgerührt. Man engte ein, nahm den Rückstand in Essigester auf und extrahierte mit wässriger Natriumbicarbonatlösung. Die organische Phase wurde mit Magnesiumsulfat getrocknet und danach das Lösungsmittel im Vakuum abgezogen. Man erhielt 2,7 g Rohprodukt, das in 13 ml Essigester gelöst und unter Rühren mit einer Lösung von 1,8 g p-Toluolsulfonsäure in 13 ml Essigester versetzt wure. Nach kurzer Zeit erfolgte Kristallisation. Man rührte noch 10 Minuten nach, gab 10 ml Diethylether zu, saugte ab, wusch mit wenig Essigester und Diethylether nach und erhielt so 1,0 g Tosylat, Fp. 160°C (Zers.).

¹H-NMR (270 MHz, DMSO-d₆): δ (ppm) = 1.15 (9H, s, tert.-Butyl), 1.52 (3H, dd, CH-CH₃), 2.3 (3H, s, C₆H₅-CH₃), 3.25 (2H, s, OCH₃), 3.7 (2H, J = 20 Hz, SCH₂-), 4.2 (2H, s, CH₂OCH₃), 5.75 (2H, 2 x dd, 6-H und 7-H), 6.85 (1H, q, OCH-CH₃), 7.15 und 7.43 (4H, 2 x d, arom. H).

### Verfahrensvariante c)

### Vorstufe 1

### 7-Amino-3-methoxymethyl-3-cephem-4-carbonsäure-α-(2,2-dimethyl-propionyloxy)-ethylester

1,46 g (6 mmol) 7-Amino-3-methoxymethyl-3-cephem-4-carbonsäure wurden in 30 ml wasserfreiem Methylenchlorid suspendiert und unter Eiskühlung mit 0,9 ml (6 mmol) 1,8-Diazabicyclo-(5,4,0)-undec-7-en (DBU) versetzt. Nach 30 Minuten wurden 1,66 g (6.5 mmol) α-2,2-Dimethyl-propionsäure-jodethylester zugetropft und 2 Stunden bei Raumtemperatur nachgerührt. Man saugte vom ausgefallenen Rückstand ab und chromatographierte das Rohprodukt (SiO₂; Toluol/Essigester = 1:1).

Es wurden 1,3 g (58 %) der Titelverbindung erhalten.

### Vorstufe 2

### 7-Bromacetyl-acetamido-3-methoxymethyl-3-cephem-4-carbonsäure-α-(2,2-dimethyl-propionyloxy)-ethylester

Zu einer Lösung von 0,26 ml (3,5 mmol) Diketen in 20 ml wasserfreiem Methylenchlorid wurden bei -40°C 0,17 ml (3,5 mmol) Brom zugetropft und 30 Minuten nachgerührt. Danach wurde eine Lösung vn 1,3 g der nach Vorstufe 1 erhaltenen Verbindung und 460 mg (3,5 mmol) N-(Trimethylsilyl)-acetamid in 10 ml Methylenchlorid zugetropft und nach 30 Minuten Rühren die Lösung eingeengt. Nach Chromatographie des Rohproduktes (SiO₂; Toluol/Essigester = 1:1) erhielt man 0,9 g Produkt.

### Vorstufe 3

### 7-(2-bromacetyl-2-hydroxyimino-acetamido)-3-methoxymethyl-3-cephem-4-carbonsäure-α-carbonsäure-(2,2-dimethyl-propionyloxy)-ethylester

Eine Lösung von 0,9 g (1,7 mmol) der in Vorstufe 2 erhaltenen Verbindung in 10 ml wasserfreiem Methylenchlorid und 5 ml Essigsäure wurde -10°C gekühlt und mit 182 mg (2,6 mmol) Natriumnitrit in 1,5 ml Wasser versetzt. Nach 30-minütigem Rühren bei -5°C wurden anschließend 162 mg (2.7 mmol) Harnstoff zugefügt und 30 Minuten nachgerührt. Nach Zugabe von 10 ml Wasser wurde die organische Phase abgetrennt, mehrmals mit Wasser und gesättigter Natriumchloridlösung gewaschen, getrocknet und das Lösungsmittel abdestilliert. Man erhielt 580 mg (63 %) der Titelverbindung.

7-[2-(2-Aminothiazol-4-yl)-2-(Z)-hydroximino-acetamido]-3-methoxymethyl)-3-cephem-4-carbonsäure-α-(2,2-dimethy-propionyloxy)-ethylester.

Zu einer Lösung von 0,57 g (1 mmol) der Vorstufe 2 in 5 ml Dimethylacetamid wurden bei 5°C 92 mg (1,2 mmol) Thioharnstoff zugegeben und 1 Stunde bei Raumtemperatur gerührt.
Anschließend versetzte man die Lösung mit 50 ml einer 3 %igen
Natriumhydrogencarbonlösung und 3,8 g Natriumchlorid, saugte den sich bildenden Niederschlag ab und nahm diesen in 15 ml Essigester und 5 ml Aceton auf. Man wusch die organische Phase mit gesättigtrer Natriumchloridlösung, trocknete über Magnesiumsulfat, zog das Lösungsmittel ab, verrieb den Rückstand mit Diethylether und erhielt so 135 mg (24 %) der gewünschten Titelverbindung, die mit der nach Verfahrensvariante a) oder b) erhaltenen identisch war.

### Beispiel 1a

### Trennung der Diastereomeren von 7-[2-(2-Aminothiazol-4-yl)-2-(Z)-hydroximinoacetamido]-3-(methoxymethyl)-3-cephem-4-carbonsäure-α-(2,2-dimethylpropionyloxy)-ethylester

### Stufe 1

Reine Diastereomeren des 7-[2-(2-Tritylaminothiazol-4-yl)-2-(Z)-(1-methyl-1-methoxy)-ethoximinoacetamido]-3-(methoxymethyl)-3-cephem-4-carbonsäure-α-(2,2-dimethylpropionyloxy)-ethylesters.

Zu einer Lösung von 14,8 g (20,3 mmol) 7-[2-(2-Tritylaminothiazol-4-yl)-2-(Z)-(1-methyl-1-methoxy)-ethoxyiminoacetamido]-3-(methoxymethyl)-3-cephem-4-carbonsäure in 100 ml wasserfreiem Methylenchlorid wurden 3 ml (20.3 mmol) 1,8-Diazabicyclo-(5,4,0)-undec-7-en (DBU) getropft. Die Lösung wurde anschließend auf 0°C abgekühlt und 6,7 g (26,4 mmol) α-2,2-Dimethylpropionsäure-jodethylester zugegeben. Man rührte noch 2 Stunden bei 0°C nach, zog anschließend das Lösungsmittel im Vakuum ab und verteilte zwischen Essigester und Wasser. Die organische Phase wurde über Magnesiumsulfat getrocknet, die Lösung eingeengt und der feste Rückstand (19,1 g), der ein Gemisch der beiden Diastereomeren enthielt, chromatographiert (900 g SiO₂; Toluol/Essigester = 5 + 1; nach Elution des ersten Diastereomeren Toluol/Essigester = 4 + 1). Man erhielt auf diese Weise 2,8 g des einen Diastereomeren (RF-Wert = 0,75; Toluol/Essigeter = 1+1) und 3,2 g des anderen Diastereomeren (RF-Wert = 0,7) in einheitlicher Form.

### Stufe 2

Reine Diastereomeren des 7-[2-(2-Aminothiazol-4-yl)-2-(Z)-hydroximino-acetamido]-3-(methoxymethyl)-3-cephem-4-carbonsäure-α-(2,2-dimethyl-propionyloxy)-ethylesters.

Jedes der beiden in Stufe 1 erhaltenen Diastereomeren wurde in 10 ml Ameisensäure gelöst, 2,5 ml Wasser zugesetzt und 1 h bei Raumtemperatur gerührt. Anschließend wurde vom gebildeten Triphenylcarbinol abgesaugt und das Filtrat im Vakuum eingeengt. Der Rückstand wurde anschließend in 100 ml Essigester aufgenommen, mit gesättigter Natriumhydrogencarbonatlösung extrahiert, die organische Phase über Magnesiumsulfat getrocknet und im Vakuum abgezogen. Den Rückstand verrieb man mit Diisopropylether. Zur weiteren Reinigung kristallisierte man aus Essigsäure-n-butylester/Diisopropylether um.
- Diastereomer I:: 1,2 g aus 2,8 g Stufe 1
Fp. = ab 166°C (Zersetzung)

¹H-NMR (270 MHz, DMSO-d₆): δ (ppm) = 1.15 (9H, s, tert.-Butyl), 1.46 (3H, d, CH-CH₃), 3.19 (3H, s, OCH₃, 3.56 (2H), AR-System, J = 6 hz, S-CH₂-), 4.15 (2H, d, CH₂OCH₃), 5.21 (1H, d, J = 6 Hz, H-6), 5.83 (1H, m, H-7), 6.64 (1H, s, Thiazol-H), 6.87 (1H, q, -O-CH-O), 7.1 (2H, breites s, NH₂), 9.5 (1H, d, J = 8 Hz, -NH), 11.3 (1H, s, Oxim-H).
- Diastereomer II:: 1 g aus 32, g Stufe 1
Fp. = ab 138°C (Zersetzung)

¹H-NMR (270 MHz, DMSO-d₆): δ (ppm) = 1.15 (9H, s, tert.-Butyl), 1.47 (3H, d, CH-CH₃), 3.2 (3H, s, OCH₃), 3.54 (2H, Ab-System, J = 6 Hz, S-CH₂-), 4.13 (2H, s, CH₂-OCH₃), 5.19 (1H, d, J = 6 Hz, H-6), 5.82 (1H, m, H-7), 6.64 (1H, s, Thiazol-H), 6.93 (1H, q, -O-CH-O), 7.1 (2H, breites s, NH₂), 9.45 (1H, d, J = 8 Hz, -NH), 11.3 (1H, s, Oxim-H).

## Patentansprüche (Patentansprüche für folgende(n) Vertragsstaat(en): AT, BE, CH, LI, DE, FR, GB, IT, LU, NL, SE)

1. Diastereomeres I des 7-[2-(2-Aminothiazol-4-yl)-2-(Z)-hydroxyiminoacetamido]-3-(methoxymethyl)-3-cephem-4-carbonsäure-[α-(2,2-dimethylpropionyloxy)-ethyl]-esters der Formel I 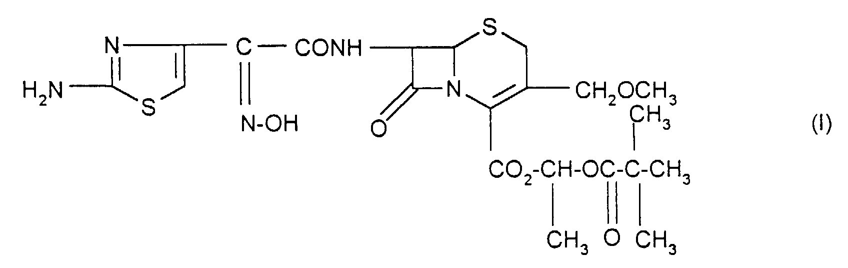 worin
die HO-Gruppe in syn-Position steht und deren physiologisch verträgliche Säureadditionssalze.

2. Verfahren zur Herstellung des Diastereomeren I gemäß Anspruch 1, dadurch gekennzeichnet, daß man
(a) eine Verbindung der Formel II 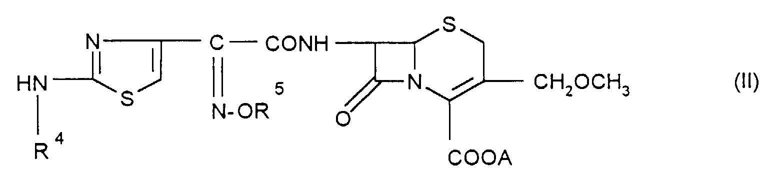 in der R⁴ für eine Aminoschutzgruppe, R⁵ eine leicht abspaltbare Gruppe und A ein Kation steht,
mit einer Verbindung der allgemeinen Formel III 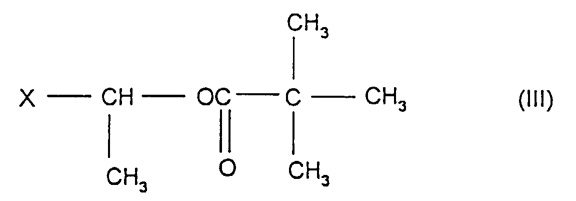 worin X für eine Abgangsgruppe steht,umsetzt zu dem Diastereomerengemisch der Verbindung der Formel IV 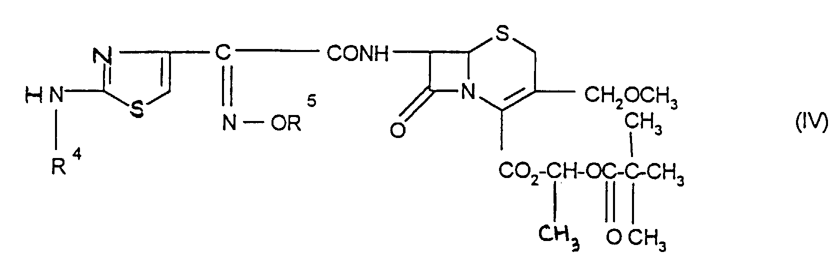 worin R⁴ und R⁵ die oben genannte Bedeutung haben, und anschließend nach an sich bekannten Methoden die Diastereomeren voneinander trennt, oder
(b) eine Verbindung der allgemeinen Formel V 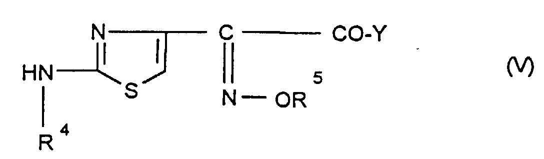 in der R⁴ und R⁵ die vorstehende Bedeutung besitzen und Y für eine aktivierende Gruppe steht, mit einer Verbindung der Formel VI 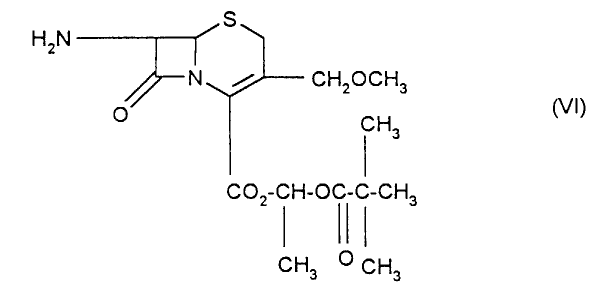 oder mit einem Salz dieser Verbindung, zu einer Verbindung der allgemeinen Formel IV umsetzt und anschließend nach an sich bekannten Methoden die Diastereomeren voneinander trennt, und
c) die Gruppen R⁴ und R⁵ vom weniger polaren Diastereomeren der Formel VI nach an sich bekannter Weise abspaltet.

3. Verfahren gemäß Anspruch 2, dadurch gekennzeichnet, daß X eine Benzolsulfonyloxy-, p-Toluolsulfonyloxy-, Methansulfonyloxy-gruppe oder Chlor, Brom oder Jod darstellt.

4. Verfahren gemäß Anspruch 2 oder 3, dadurch gekennzeichnet, daß R⁴ eine Formyl-, Chloracetyl-, Bromacetyl-, Trichloracetyl-, Benzyloxycarbonyl-, tert.-Butoxycarbonyl- oder Tritylgruppe und R⁵ eine Benzhydryl-, Trityl-, Tetrahydropyranyl- oder 1-Methoxy-1-methyl-ethylgruppe darstellt.

5. Verfahren gemäß Anspruch 2, dadurch gekennzeichnet, daß X Jod ist, R⁴ die Tritylgruppe und R⁵ die 1-Methoxy-1-methyl-ethylgruppe darstellt, und die Gruppen R⁴ und R⁵ mit Trifluoressigsäure, Ameisensäure oder verdünnter Salzsäure abgespalten werden.

6. Arzneimittel, gekennzeichnet durch einen Gehalt an dem Diastereomeren I der Formel I gemäß Anspruch 1.

7. Verfahren zur Herstellung von gegen bakteriellen Infektionen wirksamen pharmazeutischen Zubereitungen, dadurch gekennzeichnet, daß Diastereomeren I der Formel I gemäß Anspruch 1 mit pharmazeutisch üblichen Trägerstoffen oder Verdünnungsmitteln in eine pharmazeutische geeignete Verabreichungsform gebracht wird.

8. Verwendung des Diastereomeren I der Formel I gemäß Anspruch 1 zur Herstellung eines Arzneimittels mit antibiotischer Wirkung.

## Patentansprüche (Patentansprüche für folgende(n) Vertragsstaat(en): ES, GR)

1. Verfahren zur Herstellung Diastereomers I des 7-[2-(2-Aminothiazol-4-yl)-2-(Z)-hydroxyiminoacetamido]-3-(methoxymethyl)-3-cephem-4-carbonsäure-[α-(2,2-dimethyl-propionyl)-ethyl]-esters der Formel I und deren physiologisch verträglichen Säureadditionssalzen, 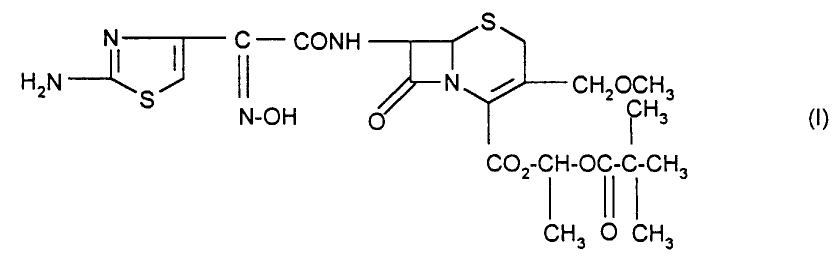 worin
die HO-Gruppe in syn-Position steht, dadurch gekennzeichnet, daß man
(a) eine Verbindung der Formel II 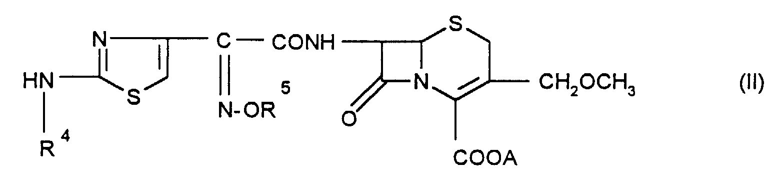 in der R⁴ für eine Aminoschutzgruppe, R⁵ eine leicht abspaltbare Gruppe und A ein Kation steht, mit einer Verbindung der allgemeinen Formel III 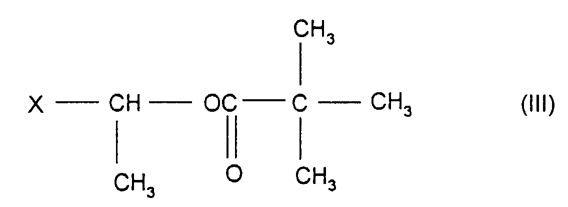 worin X für eine Abgangsgruppe steht, zu dem Ester der allgemeinen Formel IV umsetzt 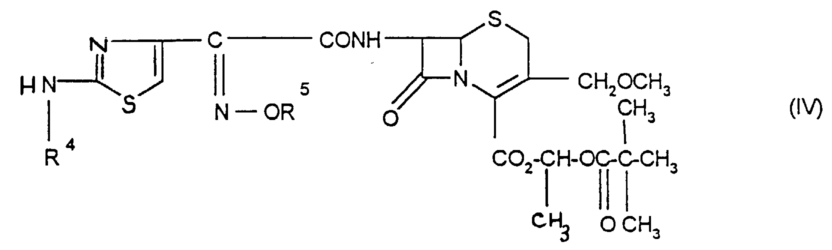 worin R⁴ und R⁵ die oben genannte Bedeutung haben, und anschließend nach an sich bekannten Methoden die Diastereomeren voneinander trennt, oder
(b) eine Verbindung der allgemeinen Formel V 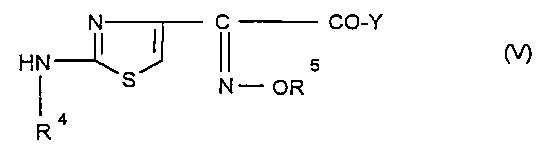 in der R⁴ und R⁵ die vorstehende Bedeutung besitzen und Y für eine aktivierende Gruppe steht, mit einer Verbindung der Formel VI 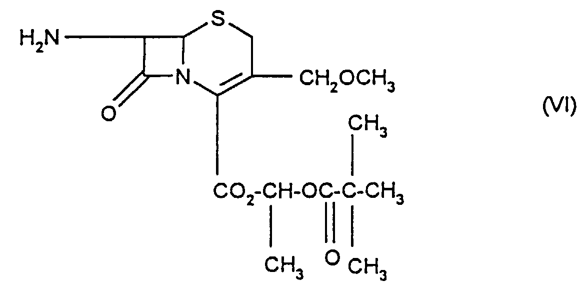 oder mit einem Salz dieser Verbindung, zu einer Verbindung der allgemeinen Formel IV umsetzt und anschließend nach an sich bekannten Methode die Diastereomeren voneinander trennt, und
c) die Gruppen R⁴ und R⁵ von weniger polaren Diastereomeren der Formel IV nach an sich bekannter Weise abspaltet.

2. Verfahren gemäß Anspruch 1, dadurch gekennzeichnet, daß X eine Benzolsulfonyloxy-, p-Toluolsulfonyloxy-, Methansulfonyloxy-gruppe oder Chlor, Brom oder Jod dartellt.

3. Verfahren gemäß Anspruch 1 oder 2, dadurch gekennzeichnet, daß R⁴ eine Formyl-, Chloracetyl-, Bromacetyl-, Trichloracetyl-, Benzyloxycarbonyl-, tert.-Butoxycarbonyl- oder Tritylgruppe und R⁵ eine Benzhydryl-, Trityl-, Tetrahydropyranyl- oder 1-Methoxy-1-methyl-ethylgruppe darstellt.

4. Verfahren gemäß Anspruch 1, dadurch gekennzeichnet, daß X Jod ist, R⁴ die Tritylgruppe und R⁵ die 1-Methoxy-1-methyl-ethylgruppe darstellt, und die Gruppen R⁴ und R⁵ mit Trifluoressigsäure, Ameisensäure oder verdünnter Salzsäure abgespalten werden.

5. Arzneimittel, gekennzeichnet durch einen Gehalt an dem Diastereomeren I der Formel I gemäß Anspruch 1.

6. Verfahren zur Herstellung von gegen bakteriellen Infektionen wirksamen pharmazeutischen Zubereitungen, dadurch gekennzeichnet, daß Diastereomeren I der Formel I gemäß Anspruch 1 mit pharmazeutisch üblichen Trägerstoffen oder Verdünnungsmitteln in eine pharmazeutisch geeignete Verabreichungsform gebracht wird.

7. Verwendung von Diastereomeren I der Formel I gemäß Anspruch 1 zur Herstellung eines Arzneimittels mit antibiotischer Wirkung.

## Claims (Claims for the following Contracting State(s): AT, BE, CH, LI, DE, FR, GB, IT, LU, NL, SE)

1. The diastereomer I of α-(2,2-dimethylpropionyloxy)ethyl 7-[2-(2-aminothiazole-4-yl)-2-(Z)-hydroxyiminoacetamido]-3-(methoxymethyl)-3-cephem-4-carboxylate of the formula I 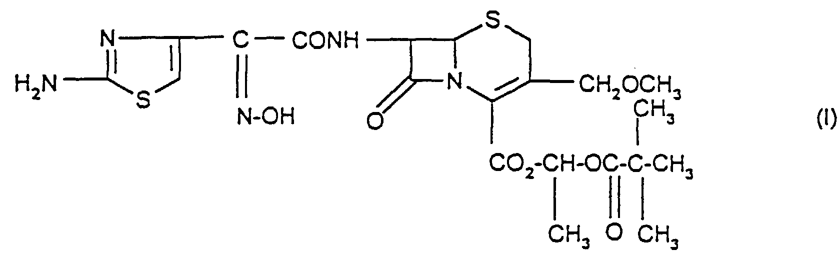 in which
the HO group is in the syn-position, or a physiologically acceptable acid-addition salt thereof.

2. The process for the preparation of the diastereomer I as claimed in claim 1, which comprises
(a) reacting a compound of formula II 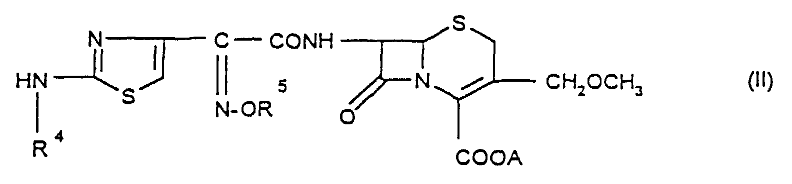 in which R⁴ is an amino-protecting group, R⁵ is a readily removable group, and A is a cation,
with a compound of the formula III 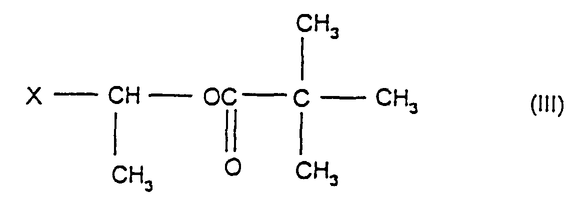 in which X is a leaving group,
to give a mixture of the diastereomers of the compound of the formula V 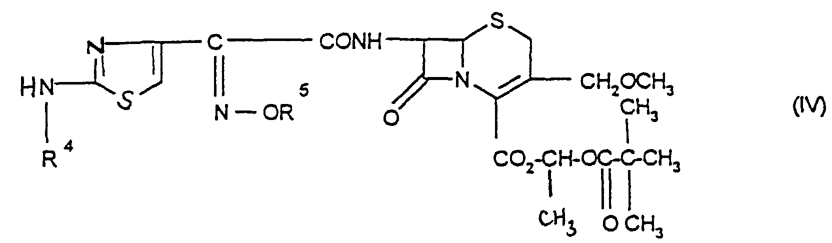 in which R⁴ and R⁵ are as defined above,
and subsequently separating the diastereomers from one another by methods known per se, or
(b) reacting a compound of the formula V 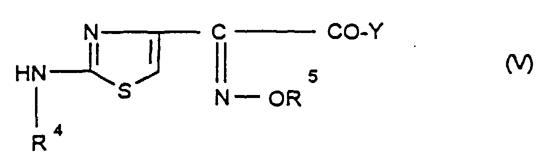 in which R⁴ and R⁵ are as defined above, and Y is an activating group,
with a compound of the formula (VI) 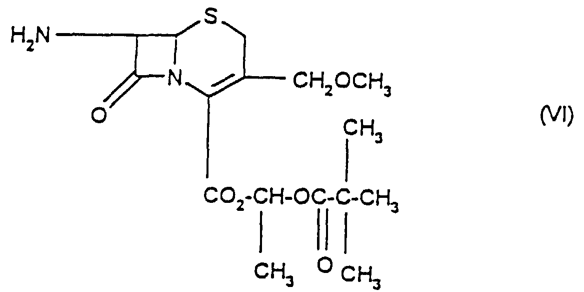 or a salt of this compound,
to give a compound of the formula IV, and subsequently separating the diastereomers from one another by methods known per se, and
c) removing the groups R⁴ and R⁵ from the less-polar diastereomer of the formula IV in a manner known per se.

3. The process as claimed in claim 2, wherein X is a benzenesulfonyloxy, p-toluenesulfonyloxy or methanesulfonyloxy group or chlorine, bromine or iodine.

4. The process as claimed in claim 2 or 3, wherein R⁴ is a formyl, chloroacetyl, bromoacetyl, trichloroacetyl, benzyloxycarbonyl, tert-butoxycarbonyl or trityl group, and R⁵ is a benzhydryl, trityl, tetrahydropyranyl or 1-methoxy-1-methylethyl group.

5. The process as claimed in claim 2, wherein X is iodine, R⁴ is the trityl group, and R⁵ is the 1-methoxy-1-methylethyl group, and the groups R⁴ and R⁵ are removed using trifluoroacetic acid, formic acid or dilute hydrochloric acid.

6. A medicament, characterized by a content of the diastereomer I of the formula I as claimed in claim 1.

7. A process for the preparation of a pharmaceutical formulation which is effective against bacterial infections, which comprises converting the diastereomer I of the formula I as claimed in claim 1 into a pharmaceutically suitable administration form using pharmaceutically customary excipients or diluents.

8. The use of the diastereomer I of the formula I as claimed in claim 1 for the preparation of a medicament having an antibiotic action.

## Claims (Claims for the following Contracting State(s): ES, GR)

1. A process for the preparation of the diastereomer I of α-(2,2-dimethylpropionyloxy)ethyl 7-[2-(2-aminothiazole-4-yl)-2-(Z)-hydroxyiminoacetamido]-3-(methoxymethyl)-3-cephem-4-carboxylate of the formula I 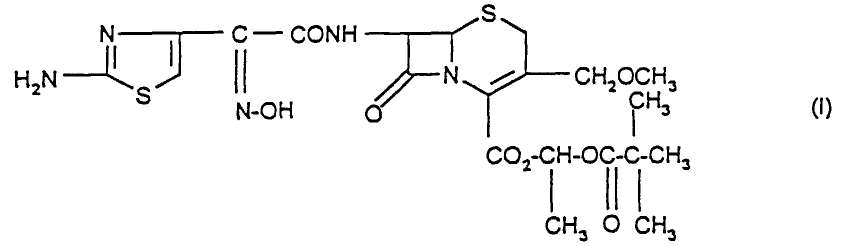 in which
the HO group is in the syn-position, or a physiologically acceptable acid-addition salt thereof
which comprises
(a) reacting a compound of formula II 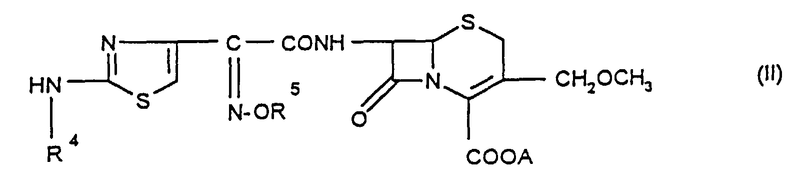 in which R⁴ is an amino-protecting group, R⁵ is a readily removable group, and A is a cation,
with a compound of the formula III 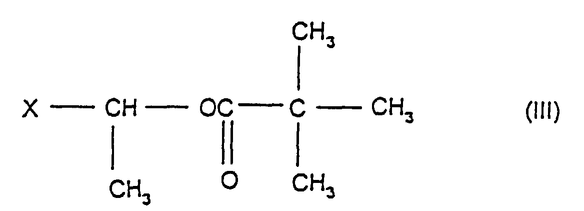 in which X is a leaving group,
to give a mixture of the diastereomers of the compound of the formula V 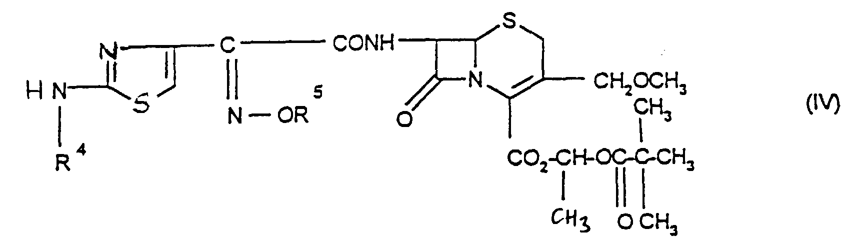 in which R⁴ and R⁵ are as defined above,
and subsequently separating the diastereomers from one another by methods known per se, or
(b) reacting a compound of the formula V 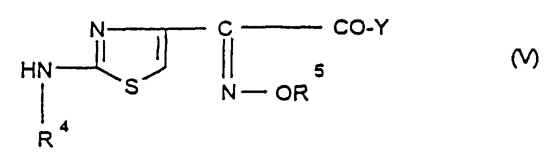 in which R⁴ and R⁵ are as defined above, and Y is an activating group,
with a compound of the formula (VI) 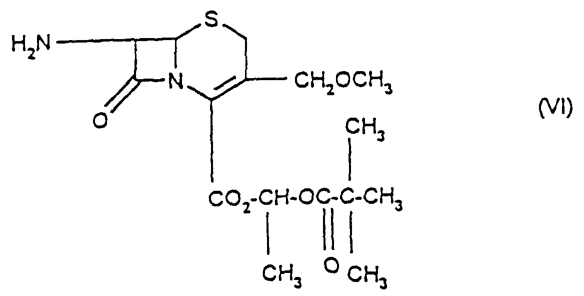 or a salt of this compound,
to give a compound of the formula IV, and subsequently separating the diastereomers from one another by methods known per se, and
c) removing the groups R⁴ and R⁵ from the less-polar diastereomer of the formula in a manner known per se.

2. The process as claimed in claim 1, wherein X is a benzensulfonyloxy, p-toluenesulfonyloxy or methanesulfonyloxy group or chlorine, bromine or iodine.

3. The process as claimed in claim 1 or 2, wherein R⁴ is a formyl, chloroacetyl, bromoacetyl, trichloroacetyl, benzyloxycarbonyl, tert-butoxycarbonyl or trityl group, and R⁵ is a benzhydryl, trityl, tetrahydropyranyl or 1-methoxy-1-methylethyl group.

4. The process as claimed in claim 1, wherein X is iodine, R⁴ is the trityl group, and R⁵ is the 1-methoxy-1-methylethyl group, and the groups R⁴ and R⁵ are removed using trifluoroacetic acid, formic acid or dilute hydrochloric acid.

5. A medicament, characterized by a content of the diastereomer I of the formula I as claimed in claim 1.

6. A process for the preparation of a pharmaceutical formulation which is effective against bacterial infections, which comprises converting the diastereomer I of the formula I as claimed in claim 1 into a pharmaceutically suitable administration form using pharmaceutically customary excipients or diluents.

7. The use of the diastereomer I of the formula I as claimed in claim 1 for the preparation of a medicament having an antibiotic action.

## Revendications (Revendications pour l'(les) Etat(s) contractant(s) suivant(s): AT, BE, CH, LI, DE, FR, GB, IT, LU, NL, SE)

1. Diastéréoisomère du 7-[2-(2-aminothiazol-4-yl)-2-(Z)-hydroxyiminoacétamido]-3-(méthoxyméthyl)-3-céphème-4-carboxylate d'[α-(2,2-diméthylpropionyloxy)éthyle] de formule I 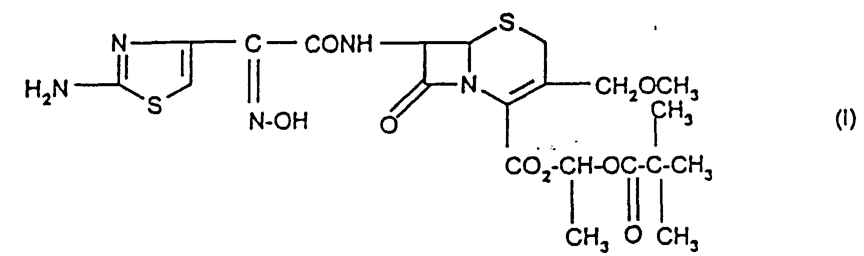 dans laquelle le groupe OH est en position *syn,*
et ses sels d'addition avec des acides physiologiquement acceptables.

2. Procédé pour la préparation du diastéréoisomère I selon la revendication 1, caractérisé en ce que
a) on fait réagir un composé de formule II 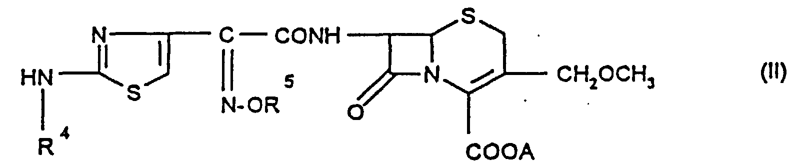 dans laquelle R⁴ représente un groupe protecteur de fonction amino, R⁵ représente un groupe aisément séparable et A représente un cation,
avec un composé de formule générale III 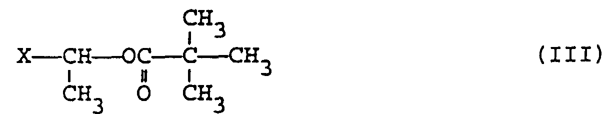 dans laquelle X représente un groupe séparable, pour aboutir au mélange de diastéréoisomères du composé de formule IV 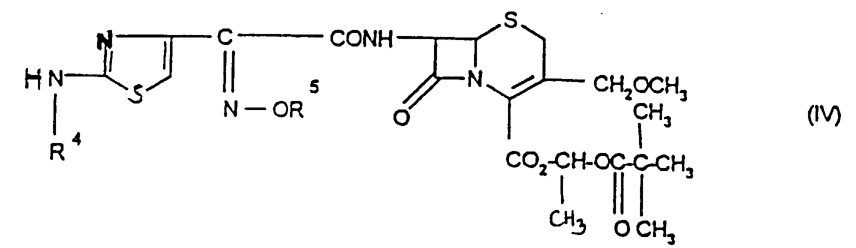 dans laquelle R⁴ et R⁵ ont les significations données plus haut, et ensuite on sépare l'un de l'autre les diastéréoisomères selon des méthodes connues en soi, ou
b) on fait réagir un composé de formule générale V 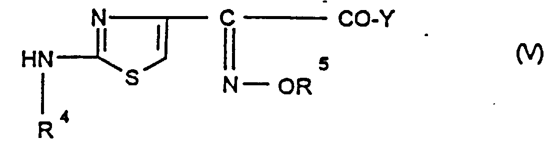 dans laquelle R⁴ et R⁵ ont les significations précédentes et Y représente un groupe activant,
avec un composé de formule VI 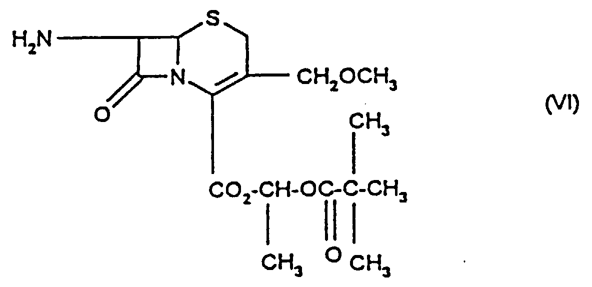 ou avec un sel de ce composé, pour aboutir à un composé de formule générale IV, et ensuite on sépare l'un de l'autre les diastéréoisomères selon des méthodes connues en soi, et
c) on élimine de façon connue en soi les groupes R⁴ et R⁵ du diastéréoisomère de formule IV le moins polaire.

3. Procédé selon la revendication 2, caractérisé en ce que X représente le groupe benzènesulfonyloxy, p-toluènesulfonyloxy, méthanesulfonyloxy ou un atome de chlore, brome ou iode.

4. Procédé selon la revendication 2 ou 3, caractérisé en ce que R⁴ représente le groupe formyle, chloracétyle, bromoacétyle, trichloracétyle, benzyloxycarbonyle, tert-butoxycarbonyle où trityle, et R⁵ représente le groupe benzhydryle, trityle, tétrahydropyrannyle ou 1-méthoxy-1-méthyléthyle.

5. Procédé selon la revendication 2, caractérisé en ce que X est un atome d'iode, R⁴ représente le groupe trityle et R⁵ représente le groupe 1-méthoxy-1-méthyléthyle, et les groupes R⁴ et R⁵ sont éliminés à l'aide d'acide trifluoroacétique, d'acide formique ou d'acide chlorhydrique dilué.

6. Médicament, caractérisé par une teneur en le diastéréoisomère I de formule I selon la revendication 1.

7. Procédé pour la préparation de compositions pharmaceutiques actives contres des infections bactériennes, caractérisé en ce que l'on met sous une forme pharmaceutique appropriée le diastéréoisomère I de formule I selon la revendication 1, avec des véhicules ou diluants pharmaceutiquement usuels.

8. Utilisation du diastéréoisomère I de formule I selon la revendication 1, pour la fabrication d'un médicament à activité antibiotique.

## Revendications (Revendications pour l'(les) Etat(s) contractant(s) suivant(s): ES, GR)

1. Procédé pour la préparation du diastéréoisomère du 7-[2-(2-aminothiazol-4-yl)-2-(Z)-hydroxyiminoacétamido]-3-(méthoxyméthyl)-3-céphème-4-carboxylate d'[α-(2,2-diméthylpropionyloxy)éthyle] de formule I 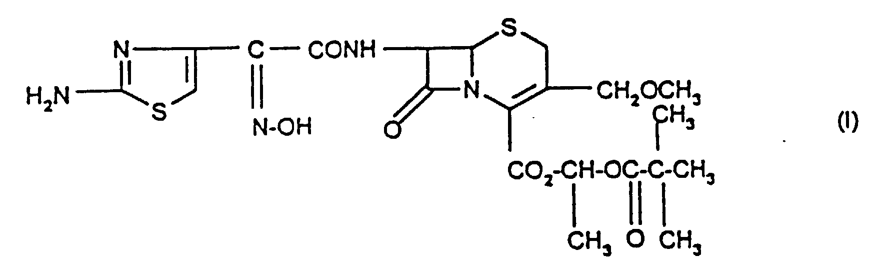 dans laquelle le groupe OH est en position *syn*,
et de ses sels d'addition avec des acides physiologiquement acceptables, caractérisé en ce que
a) on fait réagir un composé de formule II 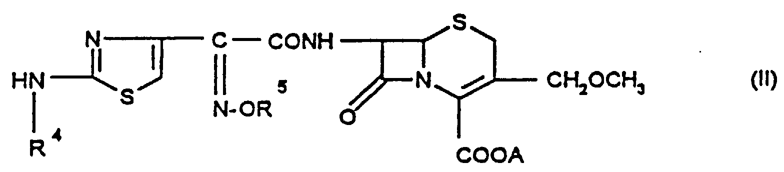 dans laquelle R⁴ représente un groupe protecteur de fonction amino, R⁵ représente un groupe aisément séparable et A représente un cation,
avec un composé de formule générale III 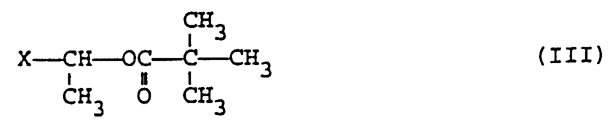 dans laquelle X représente un groupe séparable, pour aboutir au mélange de diastéréoisomères du composé de formule IV 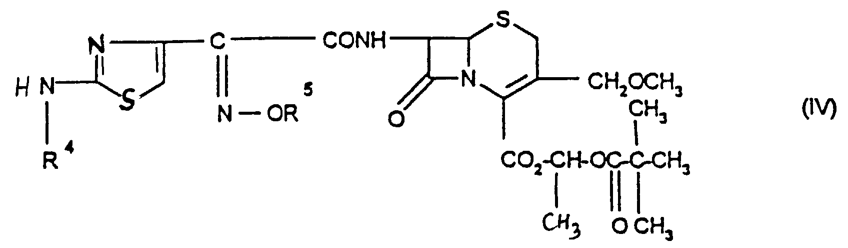 dans laquelle R⁴ et R⁵ ont les significations données plus haut, et ensuite on sépare l'un de l'autre les diastéréoisomères selon des méthodes connues en soi, ou
b) on fait réagir un composé de formule générale V 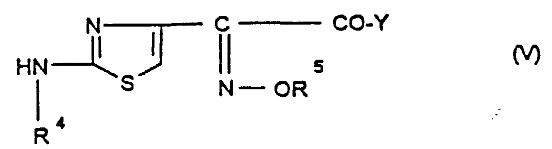 dans laquelle R⁴ et R⁵ ont les significations précédentes et Y représente un groupe activant,
avec un composé de formule VI 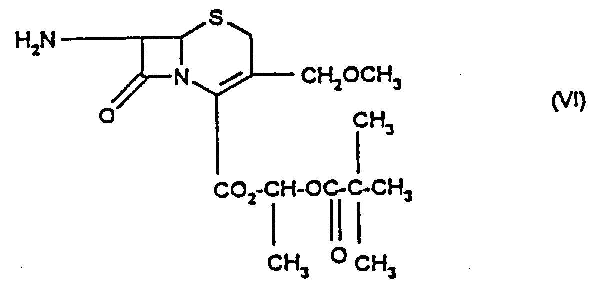 ou avec un sel de ce composé, pour aboutir à un composé de formule générale IV, et ensuite on sépare l'un de l'autre les diastéréoisomères selon des méthodes connues en soi, et
c) on élimine de façon connue en soi les groupes R⁴ et R⁵ du diastéréoisomère de formule IV le moins polaire.

2. Procédé selon la revendication 1, caractérisé en ce que X représente le groupe benzènesulfonyloxy, p-toluènesulfonyloxy, méthanesulfonyloxy ou un atome de chlore, brome ou iode.

3. Procédé selon la revendication 1 ou 2, caractérisé en ce que R⁴ représente le groupe formyle, chloracétyle, bromoacétyle, trichloracétyle, benzyloxycarbonyle, tert-butoxycarbonyle ou trityle, et R⁵ représente le groupe benzhydryle, trityle, tétrahydropyrannyle ou 1-méthoxy-1-méthyléthyle.

4. Procédé selon la revendication 1, caractérisé en ce que X est un atome d'iode, R⁴ représente le groupe trityle et R⁵ représente le groupe 1-méthoxy-1-méthyléthyle, et les groupes R⁴ et R⁵ sont éliminés à l'aide d'acide trifluoroacétique, d'acide formique ou d'acide chlorhydrique dilué.

5. Médicament, caractérisé par une teneur en le diastéréoisomère I de formule I selon la revendication 1.

6. Procédé pour la préparation de compositions pharmaceutiques actives contres des infections bactériennes, caractérisé en ce que l'on met sous une forme pharmaceutique appropriée le diastéréoisomère I de formule I selon la revendication 1, avec des véhicules ou diluants pharmaceutiquement usuels.

7. Utilisation du diastéréoisomère I de formule I selon la revendication 1, pour la fabrication d'un médicament à activité antibiotique.
